(19) Europäisches Patentamt
European Patent Office
Office européen des brevets

(11) Publication number: **0 211 630 B1**

(12) **EUROPEAN PATENT SPECIFICATION**

(45) Date of publication of patent specification: **24.07.91**

(51) Int. Cl.5: **C08G 18/10**, C08G 18/32, C08G 18/76

(21) Application number: **86305934.1**

(22) Date of filing: **01.08.86**

# (54) Liquid organic polyisocyanate compositions containing urea and/or biuret structures.

(30) Priority: **09.08.85 US 764435**

(43) Date of publication of application:
**25.02.87 Bulletin 87/09**

(45) Publication of the grant of the patent:
**24.07.91 Bulletin 91/30**

(84) Designated Contracting States:
**AT BE CH DE FR GB IT LI NL SE**

(56) References cited:
**EP-A- 0 012 970**
**EP-A- 0 135 867**
**US-A- 3 441 588**
**US-A- 3 976 622**
**US-A- 4 136 241**

(73) Proprietor: **ICI AMERICAS INC.**
**Concord Pike & New Murphy Road**
**Wilmington Delaware 19897(US)**

(72) Inventor: **Gilis, Herbert**
**4706 Apple Lane**
**West Deptford New Jersey 08066(US)**

(74) Representative: **Leherte, Georges Maurice Lucien Marie et al**
**Imperial Chemical Industries PLC Legal Department: Patents P.O. Box 6 Bessemer Road**
**Welwyn Garden City Herts. AL7 1HD(GB)**

**EP 0 211 630 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to liquid organic polyisocyanate compositions containing urea and/or biuret structures and to methods for preparing said composition.

Because of the advantageous effects which can be provided by the introduction of urea and/or biuret into polyurethanes, it is well known to modify organic polyisocyanates by the incorporation therein of urea and/or biuret groups and then to use the modified isocyanates in polyurethane manufacture.

Urea group modified isocyanates may be obtained by reacting organic polyisocyanates with a stoichiometric deficiency of water or a mono- or polyamine. Under the influence of heat, the ureas can then react with further polyisocyanates to form biurets. In many cases, however, the biuret-modified polyisocyanates, which are usually homogeneous solutions, are difficult to produce because the initially formed ureas are insoluble solids which react further only with difficulty.

In order to overcome this problem, it has been proposed in U.S. Patent No. 3441588 to react the polyisocyanates with a polyether diamine and it is said that biuret prepolymers are conveniently prepared without the undesirable formation of solid urea precipitates.

It has now surprisingly been found that isocyanate compositions which contain significant quantities of urea and/or biuret terminated structures may be prepared quite conveniently by rapid mixing of certain blocked or "retarded" polyamines with polyisocyanates. The reactions may be performed at low temperature (i.e. ambient, or slightly above). When appropriate blocking or retarding agents are used, the functionalities of the reagents (amine and/or isocyanate) may be higher than 2. The use of high-functionality reagents, either individually or in combination, does not necessitate any increase in the severity of the reaction condition (i.e. the temperature of reaction). This represents a very significant improvement, in processing convenience, over prior art methods. Modified isocyanate compositions which have previously been inaccessible or impractical may now be prepared with surprising ease. Most of the modified isocyanate (even the highest functionality combinations) exhibit surprising homogeneity and stability at ambient temperatures. The compositions are generally clear, free of solids, and low in viscosity.

Accordingly, the invention provides a modified organic isocyanate composition containing urea and/or biuret groups, and/or salts of these groups, prepared by reacting a blocked amine composition and a polyisocyanate composition, said blocked amine composition being the reaction product of:

(a) a primary and/or secondary amino-terminated polyether having an average amine functionality between 2 and 6, an average total active hydrogen functionality of not more than 10.0 and an amine equivalent weight of above 100, and

b. a reactivity modifying agent selected from proton acids, Lewis acids, silylating agents, metallating agents and alkylating agents, and said polyisocyanate composition comprising organic polyisocyanates having a reactive isocyanate functionality of at least 1.0, the reaction between said blocked amine and polyisocyanate compositions being conducted so as to provide an excess of isocyanate and/or isothiocyanate groups over total active hydrogens in the blocked amine composition of at least 1.5 : 1.0.

The primary and/or secondary amino terminated polyethers suitable for use in preparing the modified isocyanate composition have been described in US Patent No 3441588. Particular mention may be made of amino terminated polyethers based on ethylene oxide, propylene oxide, tetrahydrofuran and mixtures thereof, especially those having molecular weights in the range of 500 to 8000.

Examples of commercial amine terminated polyethers which are preferred for use in the instant invention include Jeffamine D-4000, a 4000 molecular weight primary amine terminated polypropylene oxide diamine; Jeffamine D-2000, a 2000 molecular weight primary amine terminated polypropylene oxide diamine; Jeffamine T-5000 , a 5000 molecular weight primary amine terminated polypropylene oxide triamine; Jeffamine T-3000, a 3000 molecular weight primary amine terminated polypropylene oxide triamine; Jeffamine ED-2001, a 2000 molecular weight primary amine terminated polyoxypropylene polyoxyethylene copolymer diamine; and Jeffamine ED-600, a 600 molecular weight primary amine terminated polyoxypropylene polyoxyethylene copolymer diamine; or mixtures thereof.

Suitable blocking or retarding, or "reactivity modifying" agents, for use with the aforementioned amines, include any anhydrous proton acid. Particularly suitable proton acids include hydrohalic acids such as hydrofluoric, hydrochloric, hydrobromic, or hydroiodic acids; lower molecular weight carboxylic acids such as acetic acid, propionic acid, benzoic acid, adipic acid, terephthalic acid, trifluoroacetic acid, trichloroacetic acid, carbonic acid, hydroxyacetic acid, acrylic acid, methacrylic acid, oxalic acid, malonic acid, fumaric acid, succinic acid and mixtures thereof; sulfonic acids such as methanesulfonic acid, benzenesulfonic acid, toluenesulfonic acid, trifluoromethane sulfonic acid, polystyrene sulfonic acid(s), and mixtures thereof; phosphonic acids such as phenylphosphonic acid, methylphosphonic acid and butylphosphonic acid. In some instances it may be acceptable to use the mineral oxyacids such as sulfuric acid, boric acid, nitric

acid, nitrous acid, sulfurous acid, phosphoric acid, arsenic acid, silicic acid, provided that these are substantially free of water and can be made compatible with the amine composition. In some instances it may be possible to employ blocking or retarding agents which are essentially aprotic but which will form labile complexes with the amine. These species include $CS_2$, $CO_2$, $SO_2$, $SO_3$; and Lewis acids such as inorganic or organic salts of magnesium, calcium, aluminium, iron, manganese, cobalt, zinc, lithium or boron. Some specific examples of the Lewis acidic materials include zinc propionate, calcium sulfate and hydrates thereof, magnesium acetate and hydrates thereof, magnesium chloride and its hydrates, triphenylboron, hydrated alumina and ferric chloride. Finally, it is in many instances possible to use blocking or retarding agents which are essentially permanent in nature, in the sense that their action results in the conversion of primary amines into slower reaction. These permanent blocking agents will remain associated with the nitrogen atoms of the amine even after these nitrogen atoms have been reacted with isocyanates - to form the urea and/or biuret modified compositions of the invention. Such permanent blocking agents include silylating agents such as trimethylsilylchloride, triethylsilyl bromide, trimethylsilylnitrile, trimethylsilylazide, t-butyldimethylsilyl chloride, trimethylsilyl imidazole and trimethylsilane; various organic alkylating agents such as t-butyl chloride, acrylonitrile, acrylamide, methyl methacrylate, dimethyl fumurate, butyl acrylate, isopropyl cinnamate, diphenylmethyl chloride, triphenylmethyl chloride, 1-chloroethylbenzene, 1-bromopropylbenzene and neopentyl bromide, and mixtures of such materials. It may, in some instances, be acceptable to use organometallic blocking agents such as trimethylgermanyl chloride, tributylstannyl chloride, triethyl borate, tri-n-butyl silicate, dicyclopentadienyltitanium dichloride, dicyclopentadienylniobium trichloride, mercuric acetate, and similar materials.

The choice of a blocking agent will depend upon a large number of factors, such as cost, availablility, solubility in the amine, the degree of difficulty involved in preparing the blocked amine, storage stability of the blocked amine, etc. In all cases, however, it is critical that the blocking or retarding agent be chosen so as to reduce the reactivity of the amine towards isocyanates, without actually preventing this reaction. The blocked amines must eventually react to form urea and/or biuret structures, in the presence of organic isocyanates. Accordingly, blocking agents or conditions which result in the formation of tertiary amine or quaternary ammonium species must be avoided because these types of amine derivatives cannot form urea and/or biuret species, in the presence of isocyanates. When alkylating or metallating agents are used, the agent itself and the reaction conditions must be chosen in such a way as to limit polyalkylation or polymetallation of the amine nitrogen atoms. It is, therefore, preferred that the number of reactive equivalents of alkylating or metallating agents used should be less than or equal to the number of equivalents of reactive primary amines present in the substrate amine(s). It is preferred, further, that the alkylating or metallating agents be chosen so as to provide, subsequent to reaction with the amine, a degree of steric hinderance in order to inhibit a second alkylation or metallation at the same position(s). Alkylating or metallating agents which provide steric bulk at the site of reaction are greatly preferred over those which do not. Bulky agents such as trimethylsilylnitrile are preferred over smaller alkylating agents such as methyl iodide. When labile blocking agents are used, such as the acids described hereinabove, it is preferred that the number of reactive equivalents of blocking agents used be less than or equal to the number of reactive equivalents of primary and secondary amines present in the amine substrate composition. If an excess of the labile blocking agent is used, relative to the quantity of amine groups present, then it is preferred that some provision should be made for removal or neutralaztion of the excess blocking agent subsequent to the blocking reaction.

When choosing blocking agents, whether permanent or labile, the compatibility of the blocking agent and/or the blocked amine and/or the ultimate decomposition products of the blocking agent with the isocyanate substrate composition must be allowed for. In particular, it is greatly preferred that the storage stability and the ultimate reactivity of the urea and/or biuret modified isocyanate compositions of the invention not be compromised by impurities introduced from the blocking operation. It is further preferred that these impurities do not result in the prolonged evolution of toxic or obnoxious by-products from the urea and/or biuret modified isocyanate compositions. The choice of blocking agent is therefore partly dependent upon the properties or characteristics of the final urea and/or biuret modified compositions, and of polymeric compositions derived therefrom.

According to a preferred embodiment of the invention, the blocking agent and the amine composition are combined and mixed in appropriate amounts, along with any appropriate catalysts, in a chemical reactor and heated to a temperature and pressure which are sufficient to promote the desired blocking reaction. The resulting mixture is agitated until the reaction has reached the desired degree of completion. According to the most preferred embodiments, the "chemical reactor" is simply a static (in-line) mixer or an impingement mixing apparatus into which the reagent streams are fed at a rate, temperature, pressure, and weight ratio which is most appropriate for the desired blocking reaction. Under this, most preferred,

processing arrangement, the need for bulky processing equipment and for lengthy synthetic operations is largely eliminated. If necessary or desired, however, for example when compatibility between the blocking agent and the amine composition is poor, the reaction may be performed in the presence of a solvent.

Polyisocyanate compositions which may be used in preparing the isocyanate compositions of the invention include the aliphatic and aromatic polyisocyanates already known in the polyurethane art.

Suitable aliphatic polyisocyanates include ethylene, hexamethylene and dicyclohexylmethane diisocyanates. Suitable aromatic polyisocyanates include toluene and diphenylmethane diisocyanates. The preferred polyisocyanate is 4,4'-diphenylmethane diisocyanate which may be used as the pure compound or in a liquid modified form, for example a prepolymer or uretonimine-modified MDI. Other useful polyisocyanates include mixtures of 4,4'-MDI with the 2,4'and 2,2'-isomers as well as crude products containing MDI together with higher functionality polymethylene polyphenyl polyisocyanates.

Examples of commercial polyisocyanates include Rubinate-44; Rubinate LF-168; Rubinate XI-208; experimental diisocyanate composition 4397-49 (approximately an 80:20 mole/mole blend of 4,4'- diphenylmethane diisocyanate with 2,4' - diphenylmethane diisocyanate, which also contains minor amounts of 2,2'-diphenylmethane diisocyanate); or mixtures of the above; all from Rubicon Chemicals Inc. Of course the term polyisocyanate also includes quasi-prepolymers of polyisocyanates with polyols. Examples of such materials are Rubinate LF-179 and Rubinate LF-167 (prepolymer modified MDI products); also available from Rubicon Chemicals Inc.

The urea and/or biuret modified isocyanate composition of the invention may be prepared by reacting the blocked amine composition and the polyisocyanate composition in the presence or absence of solvents. Suitable solvents include benzene toluene, chlorobenzene, o-dichlorobenzene, butyl acetate, ethylene glycol monomethyl ether acetate, methyl ethyl ketone, chloroform and methylene chloride.

Preferably, the reaction is performed in the absence of solvents in a static (in-line) mixer or an impingement mixing apparatus into which the reagent streams are fed at a rate, temperature, pressure and weight ratio appropriate for preparing the desired compositions.

The reaction temperature may be as high as 200 °C at pressures of up to 1000 atmospheres although it is not generally necessary to exceed temperatures of 100 °C or pressures of 100 atmospheres. The preferred temperature range is between 20 and 50 °C at pressures of from 1 to 10 atmospheres. Generally, the higher reaction temperature and the longer the residence time at a given temperature, the greater the extent of biuret formation. A low level of functionality and hence a low level of biuretisation is generally desirable when the isocyanate compositions are intended for use in the manufacture of elastomeric or flexible materials. For the production of, for example, rigid foam, a higher level of biuretisation may be desirable making it appropriate to use more sever conditions for the reaction between the blocked amine composition and the polyisocyanate composition.

The urea and/or biuret modified isocyanate compositions of the invention is prepared by using an excess of isocyanate groups over active hydrogen groups of at least 1.5:1 on an equivalent basis. In the more preferred embodiments of the invention, however, the ratio of isocyanate groups to the sum total of active hydrogen atom containing groups used to prepared the modified isocyanate compositions of the invention is at least 3:1, on an equivalents basis. In the most preferred embodiments, the ratio of isocyanate groups to the sum total of active hydrogen atom containing groups used to prepare the modified isocyanate compositions of the invention is at least 5:1, on an equivalent basis. The "active hydrogen atoms containing groups" referred to hereinabove are to be understood as the sum of the reactive (primary and secondary) amine groups present (whether free, or in "blocked" or latent form) within the blocked polyamine composition, and any additional Zerewitinoff active hydrogen groups, such as alcohols, phenols, thiols, carboxylic acids.

The modified isocyanate compositions of the invention may be used in the manufacture of polyurethane, polyurethaneurea, polyurea and polyamide materials by reaction with appropriate active hydrogen compounds under appropriate conditions. Suitable active hydrogen compounds include organic compounds containing a plurality of amino, hydroxy, carboxy or thiol groups. Such compounds generally have molecular weights of from 200 to 10,000, preferably between 500 and about 5000.

Amino-containing compounds which may be reacted with the isocyanate compounds include those already described herein, for example amino-terminated polyether.

Hydroxy compounds which may be reacted with the isocyanate compositions include the polyether and polyester polyols already known for use in the manufacture of polyurethanes. Polyesteramides and polyacetals may also be employed. The polyethers particularly include the oxyalkylation products of active hydrogen compounds, for example, products obtained by reacting ethylene oxide and/or propylene oxide with polyols or polyamines. Suitable polyesters include the reaction products of glycols and other polyols with aliphatic and/or aromatic dicarboxylic acids.

The reaction between modified isocyanate compositions and the active hydrogen compounds may be performed under conditions that have been fully described in the polyurethane literature. Thus, the reaction may be performed in the presence, as appropriate, of blowing agents, for example, water and/or trichlorofluoromethane, catalysts, for example, tertiary amines and/or tin compounds, surfactants, for example, siloxane-oxyalkylene copolymers, chain-extenders, for example, glycols or diamines, pigments, fillers, flame-retardants.

The products obtained by reacting the isocyanate compositions with active hydrogen compounds may take the form of flexible or rigid foams, solid elastomers, coatings and the like. Where appropriate, it is preferred to use the reaction injection moulding (RIM) process.

The invention is illustrated by the following Examples.

Glossary of Terms:

Polyamine A: A linear 2000 molecular weight primary amine terminated polyoxypropylene diamine, which is commercially available from Texaco Chemical Corp. as "Jeffamine D-2000®".

Polyamine B: A linear 400 molecular weight primary amine terminated polyoxypropylene diamine, which is commercially available from Texaco Chemical Corp. as "Jeffamine D-400®".

Polyamine C: A linear 4000 molecular weight primary amine terminated polyoxypropylene diamine, which is commercially available from Texaco Chemical Corp. as "Jeffamine D-4000®".

Polyamine D: A glycerol initiated 5000 molecular weight primary amine terminated polyoxypropylene triamine, which is commercially available from Texaco Chemical Corp. as "Jeffamine T-5000®".

Polyamine E: A glycerol initiated 3000 molecular weight primary amine terminated polyoxypropylene triamine, which is commercially available from Texaco Chemical Corp. as "Jeffamine T-3000®".

Polyamine F: A linear 2000 molecular weight polyoxyethylene polyoxypropylene copolymer based diprimary diamine in which about 94 mole percent of the total oxyalkylene units in the chain are oxyethylene units and the remaining 8 mole percent are oxypropylene units. This substance is commercially available from Texaco Chemical Corp. as "Jeffamine ED-2001®".

Polyamine G: A linear 600 molecular weight primary amine terminated polyoxyethylene polyoxypropylene copolymer diamine in which about 77 mole percent of the total oxyalkylene units in the chain are oxyethylene units and the remaining 23 mole percent are oxypropylene units. This substance is commercially available from Texaco Chemical Corp. as "Jeffamine ED-600®".

Isocyanate I: Pure 4,4'-diphenylmethane diisocyanate, containing not more than 2% by weight of the 2,4'-diphenylmethane diisocyanate isomer. This material is commercially available from Rubicon Chemicals Inc.

Isocyanate J: A mixture consisting of approximately 80% by weight of 4,4'-diphenylmethane diisocyanate and about 20% by weight 2,4'-diphenylmethane diisocyanate. Commercially available from Rubicon Chemicals Inc.

Isocyanate K: A uretonimine modified derivative of Isocyanate I, having a free isocyanate (NCO) content of 29.3% by weight and a number averaged functionality of about 2.15. This substance is commercially available from Rubicon Chemicals Inc., as "Rubinate LF-168®".

Isocyanate L: A uretonimine modified derivative of Isocyanate J, having a free isocyanate (NCO) content of 31.0% by weight and a number averaged functionality of about 2.08. This substance is commercially available from Rubicon Chemicals Inc. as "Rubinate X1-208®".

Isocyanate M: Phenyl isocyanate.

Isocyanate N: A urethane pseudoprepolymer modified derivative of Isocyanate I which is prepared by reacting Isocyanate with a mixture of low molecular weight glycols. This substance has a free isocyanate (NCO) content of about 23.0% by weight, a number averaged functionality of 2.00, and is commercially available from Rubicon Chemicals Inc. as "Rubinate LF-179®".

Isocyanate 0: A blend consisting of 50% by weight Isocyanate K and 50% by weight Isocyanate N. This substance is commercially available from Rubicon Chemicals Inc. as "Rubinate LF-167®".

Isocyanate P: A mixture consisting of about 70% by weight of 4,4'diphenylmethane diisocyanate and about 30% 2,4-diphenylmethane diisocyanate.

Isocyanate Q: A mixture consisting of about 80% 2,4-toluene diisocyanate and about 20% 2,6-toluene diisocyanate. This substance is commercially available from Rubicon Chemicals Inc. as "Rubinate TDI®".

Isocyanate R: Is the meta isomer of tetramethylxylene diisocyanate. This material is commercially available from American Cyanamide Corp. as "m-TMXDI®".

Isocyanate S: Polymeric polyphenylmethane polyisocyanate, or "crude MDI". A mixture of various isomers of diphenylmethane diisocyanate with a series of higher functionality methylene bridged polyphenylmethane

isocyanates, in a ratio of about 1:1 by weight. This substance has a number averaged isocyanate functionality of about 2.75 and a functional group (NCO) concentration of about 31.5% by weight. It is commercially available from Rubicon Chemicals Inc. as "Rubinate M®".

Isocyanate T: A modified version of Isocyanate S, in which the diphenylmethane diisocyanate isomer content has been increased by blending with Isocyanate J. This substance has a number averaged isocyanate functionality of about 2.40 and a functional group (NCO) concentration of about 31.5% by weight. It is commercially available from Rubicon Chemicals Inc. as "Rubinate XI-182®".

Preparations 1-10 are intended to illustrate convenient methods for preparing the blocked, "retarded", or "reactivity modified" polyamine compositions which are useful in the practice of the instance invention.

Preparation 1

A flask was charged with 350 g of Polyamine D. The flask, equipped with a thermometer, overhead stirrer, and an inert gas inlet, was gently and continuously purged with a stream of dry nitrogen. A gas sparging tube was inserted into the flask, with the outlet well below the level of liquid (Polyamine D). The liquid was gently agitated and sparged, beneath its surface, with anhydrous hydrochloric acid (HC1) in large excess. The excess HC1 was swept out of the flask via the nitrogen purge. The HC1 sparging was initiated at ambient temperatures (20-25 °C.) and continued until after the reaction exotherm had peaked and subsided. The total reaction time was 1 hr. (HC1 sparging) and the peak exotherm reached 53 °C. during this preparation. The product was a clear liquid.

Preparation 2

Following the procedure analogous to that described in Preparation 1, a 350 g sample of the anhydrous hydrochloride salt of Polyamine E was prepared. The total sparging time was approximately 1 hr. The product was a clear liquid.

Preparation 3

Following the procedure analogous to that described in Preparation 1, a 350 g sample of the anhydrous hydrochloride salt of Polyamine G was prepared. The total sparging time was approximately 1 hour. The product was a clear liquid.

Preparation 4:

Following the procedure analogous to that described in Preparation 1, a 350 g sample of the anhydrous hydrochloride salt of Polyamine C was prepared. The total sparging time was approximately 1 hr. The product was a clear liquid.

Preparation 5:

A flask, equipped with thermometer, overhead stirrer, and a purge of dry nitrogen, was charged with 300 g of Polyamine C. Adipic acid, 11.0 g., was then suspended in the liquid polyamine. The resulting suspension was agitated and heated until all the solids dissolved. The system became clear and homogenous at 85 °C. The product remained homogeneous at ambient temperatures.

Preparation 6:

Following a procedure analogous to that described in Preparation 5, a 300 g sample of Polyamine D was modified with 13.16 g of adipic acid. The adipic acid was initially suspended in the polyamine. The system was then heated until clear (85 °C). The product was a clear homogeneous liquid at ambient temperatures.

Preparation 7:

A flask was charged with 325 g of Polyamine C. The flask, equipped with a thermometer, overhead

stirrer, and an inert gas inlet was gently and continuously purged with a stream of dry nitrogen. A gas purging tube was inserted into the flask, with the outlet well below the level of liquid (Polyamine C). The liquid was gently agitated and sparged, beneath its surface, with anhydrous carbon dioxide ($CO_2$) in large excess. The excess $CO_2$ was swept out of the flask via the nitrogen purge. The $CO_2$ sparging was initiated at 23° C. and continued until after the reaction exotherm had peaked and subsided. The total reaction time was 1 hr. ($CO_2$ sparging) and the peak exotherm reached 30 °C during this preparation. The product was a clear liquid.

Preparation 8

Following a procedure analogous to that described in Preparation 7, a 325 g sample of the carbon dioxide modified derivative of Polyamine A was prepared. The total sparging time was approximately 1 hour and the peak exotherm reached 34° C. The product was a clear liquid.

Preparation 9

A flask was charged with 283.2 g of Polyamine D. The flask, equipped with a thermometer, an overhead stirrer, and an inert gas inlet, was maintained under a slight positive pressure with dry nitrogen. The liquid Polyamine D was agitated and trimethylsilylnitrile, 16.85 g., was added steadily via syringe over a period of 2 minutes. The temperature of the reaction increased from 23° C to 31° C during the addition - due to a spontaneous exotherm. Modest gas evolution was noted. This gas was allowed to expand against the nitrogen back pressure and escape through a side outlet in the nitrogen line. The gas was vented into a fume hood. After the addition was completed, the temperature of the reaction was increased to 80° C over 13 min. and maintained at 80° C, with continued agitation, for an additional 40 minutes. The flask was then attached to a vacuum line and the pressure over the liquid mixture was reduced from ambient to approximately 1 mm.Hg. (133 Pa). Vigorous gas evolution was noted during this depressurization step. This gas was condensed in a dry ice trap. The outlet from the vacuum pump was vented into a fume hood. Gas evolution was observed to have ceased 8 minutes after the vacuum was applied. The temperature of the system was further increased to 105° C over a period of 10 minutes. Heating was the discontinued and the product was allowed to cool. Agitation was discontinued and the pressure in the flask was allowed to increase, to ambient, by introduction of dry nitrogen. The product was a clear liquid.

Preparation 10

Following a procedure closely analogous to that used in Preparation 9, a sample consisting of 100 g of Polyamine B was reacted with 49 g of trimethylsilylnitrile. The total reaction time was 83 minutes, the maximum temperature reached during the vacuum degassing stage was 100° C, and the final product was a clear liquid.

Example 1

A sample of the polyamine derivative prepared in Preparation 2 was added directly to a 90:10 (w/w) blend of Isocyanate J and K. The addition was performed at a temperature of 46° C under an atmosphere of dry nitrogen. The isocyanate blend was agitated gently throughout the addition. The addition was conducted over a period of 43 minutes. Following the addition, the pressure within the reaction vessel was reduced to approximately 1 mm.Hg. (133 Pa) and agitation was continued for 1 hour longer. Pressure within the vessel was then increased to ambient by slow introduction of dry nitrogen. Agitation was discontinued and the sample was stored under an atmosphere of dry nitrogen at ambient temperatures. The product was observed to be a clear homogeneous liquid. The product was free of solids, gels, or cloudiness after 3 weeks of storage. No solids or gels were observed at any point during the preparation. The final formulation is listed below, with each ingredient as a percent by weight of the total:

Isocyanate J = 45.0%
Isocyanate K = 5.0%
Derivative of Polyamine E (from Preparation 2) = 50.0%

Attempts to form a composition analogous to that shown under Preparation 1 according to a procedure analogous to that described in Preparation 1 but using untreated Polyamine E (instead of the derivative prepared in Preparation 2) were not successful. Solids began forming instantly when Polyamine E was added to the isocyanate blend of Example 12. Continued addition of Polyamine E resulted in a steady

buildup of gel-like solids.

Example 2

A sample of the polyamine derivative prepared in Preparation 1 was added directly to a 90:10 (w/w) blend of Isocyanate J and K. The addition was performed at a temperature of 48° C under an atmosphere of dry nitrogen. The addition was conducted over a period of 26 minutes. Following the addition, the pressure within the reaction vessel was reduced to approximately 1 mm.Hg. (133 Pa) and agitation was continued for 1 hour longer. Pressure within the vessel was then increased to ambient by slow introduction of dry nitrogen. Agitation was discontinued and the sample was stored, under an atmosphere of dry nitrogen at ambient temperatures. The product was observed to be a clear homogeneous liquid. The product was free of solids, gels, or cloudiness after 6 weeks of storage. No solids or gels were observed at any point during the preparation. The final formulation is listed below, with each ingredient as a percent by weight of the total:

Isocyanate J = 45.0%
Isocyanate K = 5.0%
Derivative of Polyamine D (from Preparation 1) = 50.0%

Attempts to form a composition analogous to that shown under Example 2 according to a procedure analogous to that described in Example 2 but using untreated Polyamine D (instead of the derivative prepared in Preparation 1) were not successful. Solids began forming instantly when Polyamine D was added to the isocyanate blend of Example 2. Continued addition of Polyamine D resulted in a steady buildup of gel-like solids.

Example 3

A sample of the polyamine derivative prepared in Preparation 3 was added directly to a sample of Isocyanate L. The procedure followed was closely analogous to that used in Examples 1 and 2, except that the addition time was 15 minutes and the vacuum treatment of the crude product was omitted. The maximum temperature reached during the processing was 50° C. A sample of the product was transferred to a dry glass jar and allowed to cool at ambient temperature for 1 hour under an atmosphere of dry nitrogen. The product was a clear liquid which was free of solids or gels. The final formulation is listed below, with each ingredient as a percent by weight of the total:

Isocyanate L = 95.0%
Derivative of Polyamine G (instead of the derivative prepared in Preparation 3) were not successful. Solids began forming rapidly when Polyamine G was added to Isocyanate L. Continued addition resulted in a steady buildup of gel-like solids. These solids would not redissolve, even when the temperature of the heterogeneous mixture was increased to 80° C.

Example 4

A sample of the polyamine derivative of Preparation 2 was added directly to a sample of Isocyanate S. The isocyanate was agitated gently during the addition and the reaction vessel was purged continuously with dry nitrogen. The addition was conducted over less than 2 minutes at an initial temperature of 22° C. The temperature of the system increased to 42° C., due to the exotherm of the reaction. No solids or gels were observed during or after the addition, but the viscosity of the formulation increased to the point where further agitation became labored. The formulation is listed below, with each ingredient as percent by weight:

Isocyanate S = 50.0%
Derivative of Polyamine E (from Preparation 2) = 50.0%

In order to reduce the viscosity of the modified isocyanate composition to manageable levels, the above formulation was back blended with Isocyanate S and the temperature of the initial blend was increased momentarily to 55° C in order to facilitate the mixing. The final formulation is listed below with each ingredient as a percent by weight:

Isocyanate S = 75.0%
Derivative of Polyamine E (from Preparation 2) = 25.0%

This final product was transferred to a dry glass jar and stored at ambient temperature under an atmosphere of dry nitrogen. The product was observed to be a clear homogeneous liquid, free of solids or gels, after 7 days of storage. No solids, gels, or other signs of in homogenity were observed at any point during the preparation or storage of this product.

Attempts to form a composition analogous to that shown under Example 4 according to a procedure analogous to that described in Example 4, but using untreated Polyamine E (instead of the derivative prepared in Preparation 2) were not successful. Solids began forming when the first drop of Polyamine E was added to Isocyanate S. Continued addition of Polyamine E resulted in a steady buildup of gel-like solids.

Example 5

A sample of the polyamine derivative of Preparation 10 was added directly to a sample of Isocyanate L. The isocyanate was agitated during the addition and the reaction vessel was purged continuously with a stream of dry nitrogen. The addition was conducted over 0.5 hours at a temperature of 50 °C. The formulation thus prepared is listed below, with each ingredient as percent by weight:

Isocyanate L = 75.0%

Derivative of Polyamine B (from Preparation 10) = 25.0%

This product, although homogeneous in bulk, was highly viscous at ambient temperatures. A sample of the product was reheated to 47 °C and back blended with Isocyanate L, with gentle agitation, to give the final formulation listed below:

Isocyanate L = 85.0%

Derivatives of Polyamine B (from Preparation 10) = 15.0%

This final product was transferred to a dry glass jar and stored at ambient temperatures under an atmosphere of dry nitrogen. The product was observed to be a clear pourable liquid and remained homogeneous in bulk after 1 month of storage.

Attempts to form a composition analogous to that shown under Example 5 according to a procedure analogous to that described in Example 5, but using untreated Polyamine B (instead of the derivative prepared in Preparation 10), were not successful. Solids began forming when the first drop of Polyamine B was introduced to Isocyanate L. Continued addition of Polyamine B resulted in a steady buildup of gellike solids. These solids would not redissolve when the temperature of the reaction was increased to 80 °C.

Example 6

A sample of the polyamine derivative prepared in Preparation 9 was added directly to a blend of Isocyanate J and K. The isocyanate blend was agitated continuously during the addition and the reaction vessel was purged with a stream of dry nitrogen. The addition was conducted over a period of 26 minutes at a temperature of 50 °C. The formulation thus prepared is listed below, with each ingredient as percent by weight:

Isocyanate J = 45.0%

Isocyanate K = 5.0%

Derivative of Polyamine D (from Preparation 9) = 50.0%

This product was clear, liquid and free of solids or gels at the conclusion of this reaction.

Example 7

A sample of the polyamine derivative prepared in Preparation 8 was rapidly poured in a well agitated sample of Isocyanate L. This addition took place over a period of not more than 5 seconds. The reaction vessel, containing the isocyanate, was continuously purged with dry nitrogen during the addition and subsequent mixing. The addition was conducted with both components at ambient temperatures. The formulation thus prepared is listed below, with each ingredient as percent by weight:

Isocyanate L = 75.0%

Derivative of Polyamine A (from Preparation 8) = 25.0%

Within about 5 seconds of the addition, vigorous foaming was observed. The system foamed to more than twice its original volume. This foaming rapidly subsided, and there remained a homogeneous liquid product. The reaction was accompanied by a mild exotherm. The maximum temperature reached during this experiment was 33 °C. The product was allowed to cool, with gentle agitation for 10 minutes and was then transferred to a dry glass jar and sealed under an atmosphere of dry nitrogen. After approximately 12 hours storage at ambient temperatures, the product was found to have a viscosity of 975 cps (.975 Pas) (at 23 °C). The product was a clear homogeneous liquid, free of solids or gels. No solids, gels, or separations were observed at any point during the preparation of this product.

Attempts to form a composition analogous to that shown under Example 7, according to the procedure

of Example 7, but using untreated Polyamine A (instead of the derivative prepared in Preparation 8), were not successful. Soft gelatinous semi-solids began forming during the addition of Polyamine A to Isocyanate L. At the conclusion of the reaction the product was clearly heterogeneous, with gelled solids evident. After approximately 12 hours storage at ambient temperatures, the product was found to have a viscosity of 3685 cps (3.685 Pas) (at 23 °C). The product was opaque, heterogeneous, and showed evidenced of bulk separation.

Example 8

A bis-urea model compound was prepared by reacting Polyamine A with Isocyanate M, so as to provide 0.99 equivalents of isocyanate (NCO) per equivalent of amine ($NH_2$) The amine was, therefore, used in slight excess. The reaction was performed by mixing the ingredients, at ambient temperatures, under an atmosphere of dry nitrogen. The product of this reaction was a clear colorless liquid which exhibited a carbonyl stretch in the infrared spectrum at 1698 $cm^{-1}$.

A model biuret compound was prepared by reacting Polyamine A with Isocyanate M so as to provide 2.02 equivalents of isocyanate groups per equivalent of $NH_2$ or, in other words, 1.01 equivalents of "NCO" per equivalent of amine hydrogen. Thus a slight excess of isocyanate was used in the preparation. The reaction was performed by initially mixing the ingredients at ambient temperature, followed by heating at 120 °C for 75 minutes and then 85 °C for 30 minutes. Under an atmosphere of dry nitrogen. The product was a clear pale yellow liquid which exhibited a principal carbonyl stretch in the infrared spectrum at 1721 $cm^{-1}$. This peak was notably absent in the spectrum of the model urea compound described hereinabove. Moreover, the starting polyamine and isocyanate used to prepare the model compounds are devoid of carbonyl species and, hence, devoid of carbonyl absorptions in the infrared (the NCO) group is, strictly speaking, an exception to this rule; but its absorption occurs above 2200 $cm^{-1}$ which places it well above the region of interest.

This particular pair of model compounds was selected because they contain key structural elements which are identical to those present in many of the modified isocyanate compositions which are compared in past examples. The structural elements are:

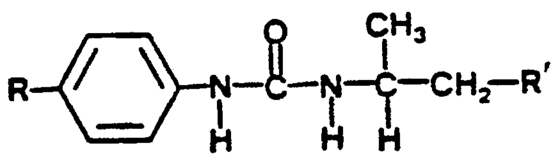

urea

and

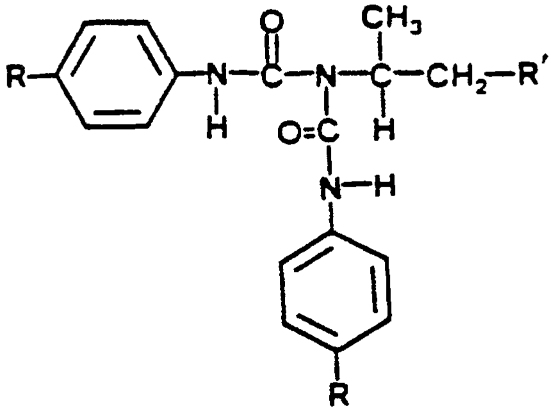

biuret

wherein R represents hydrogen or a $-CH_2$-terminated organic radical and R' represents a polyether chain. It is therefore possible to locate signals in the spectra of the modified polyisocyanate compositions which are homologous to those of the model compounds. In this way, the progress of the biuretization reaction can be followed as a function of processing conditions. The spectroscopic results can be compared to the concentration of residual isocyanate (NCO) groups in the sample. It is possible, also, to assess whether or not the blocked, or reactivity modified, polyamine composition has indeed reacted with the isocyanate substrate composition to form urea and/or biuret linkages. The presence of carbonyl signals in the IR (below 2200 $cm^{-1}$) is generally diagnostic of reaction. Infrared analyses were performed on the modified isocyanate compositions prepared in Examples 1, 2, 4, 5, 7 and 8. These analyses showed the presence of the new carbonyl species, formed in the reaction between the isocyanate and blocked polyamine.

**Claims**

**1.** A modified organic isocyanate composition containing urea and/or biuret groups, and/or salts of these groups, prepared by reacting a blocked amine composition and a polyisocyanate composition, said blocked amine composition being the reaction product of:

(a) a primary and/or secondary amino-terminated polyether having an averaged amine functionality between 2 and 6, an average total active hydrogen functionality of not more than 10.0 and an amine equivalent weight of above 100, and

(b) a reactivity modifying agent selected from proton acids, Lewis acids, silylating agents, metallating agents and alkylating agents, and said polyisocyanate composition comprising organic polyisocyanate functionality of at least 1.0

whereby said blocked amine is formed under conditions which inhibit the formation of tertiary amine of quaternary ammonium species, and the reaction between said blocked amine and polyisocyanate compositions being conducted as to provide an excess of isocyanate groups over total active hydrogens in the blocked amine composition of at least 1.5:1.0.

**2.** A modified isocyanate composition according to claim 1 wherein the amino terminated polyether has a molecular weight in the range of 500 to 8000 is based on ethylene oxide, propylene oxide, tetrahydrofuran or mixtures thereof.

**3.** A modified isocyanate composition according to claim 1 wherein the poylisocyanate composition is on includes a diphenylmethane diisocyanate.

**4.** A modified isocyanate composition according to claim 3 wherein the polyisocyanate composition is or includes a 4,4' diphenylmethane diisocyanate.

**5.** A modified isocyanate composition according to claim 4 wherein the polyisocyanate composition comprises a mixture of 4,4'-diphenylmethane diisocyanate with the 2,4'- and 2,2'-isomers.

**6.** A modified isocyanate composition according to claim 1 wherein the ratio of isocyanate group to total active hydrogens the blocked amine composition is at least 5:1.

**Revendications**

**1.** Composition d'isocyanate organique modifié contenant des radicaux urée et/ou biuret et/ou des sels de ces radicaux, préparée par réaction d'une composition d'amine bloquée et d'une composition de polyisocyanate, la composition d'amine bloquée étant le produit de réaction :

(a) d'un polyéther à radicaux amino primaires et/ou secondaires terminaux ayant une fonctionnalité amine moyenne entre 2 et 6, une fonctionnalité hydrogène actif total moyenne n'excédant pas 10,0 et un poids équivalent d'amine de plus de 100, et

(b) d'un agent modificateur de la réactivité choisi parmi les acides protiques, les acides de Lewis, les agents de silylation, les agents de métallation et les agents d'alcoylation,

et la composition de polyisocyanate comprenant une fonctionnalité polyisocyanate organique d'au moins 1,0,

tandis que l'amine bloquée est formée dans des conditions qui inhibent la formation d'espèces amine tertiaire ou ammonium quaternaire et la réaction entre les compositions d'amine bloquée et de polyisocyanate étant conduite de façon à établir un excès de radicaux isocyanate sur les hydrogènes

actifs totaux dans la composition d'amine bloquée d'au moins 1,5:1,0.

2. Composition d'isocyanate modifié suivant la revendication 1, dans laquelle le polyéther à radicaux amino terminaux a un poids moléculaire de l'intervalle de 500 à 8000 et est à base d'oxyde d'éthylène, d'oxyde de propylène, de tétrahydrofuranne ou de mélanges de ceux-ci.

3. Composition d'isocyanate modifié suivant la revendication 1, dans laquelle la composition de polyisocyanate est ou comprend un diisocyanatodiphénylméthane.

4. Composition d'isocyanate modifié suivant la revendication 3, dans laquelle la composition de polyisocyanate est ou comprend un 4,4'-diisocyanatodiphénylméthane.

5. Composition d'isocyanate modifié suivant la revendication 4, dans laquelle la composition de polyisocyanate comprend un mélange de 4,4'-diisocyanatodiphénylméthane et des isomères 2,4' et 2,2'.

6. Composition d'isocyanate modifié suivant la revendication 1, dans laquelle le rapport des radicaux isocyanate aux hydrogènes actifs totaux dans la composition d'amine bloquée est d'au moins 5:1.

**Patentansprüche**

1. Modifizierte organische Isocyanatzusammensetzung, die Harnstoff- und/oder Biuret-Gruppen und/oder Salze dieser Gruppen enthält und durch Reaktion einer desaktivierten Aminzusammensetzung und einer Polyisocyanatzusammensetzung hergestellt wurde, wobei die desaktivierte Aminzusammensetzung das Reaktionsprodukt ist von:
(a) einem Polyether mit einer mittleren Aminfunktionalität zwischen 2 und 6, einer mittleren Gesamtfunktionalität an aktivem Wasserstoff von nicht mehr als 10,0, sowie einem Aminäquivalentgewicht von über 100, dessen Endgruppen primäre und/oder sekundäre Aminogruppen sind, und
(b) einem die Reaktivität modifizierenden Agens, aus-gewählt aus Protonsäuren, Lewissäuren, Silylierungsmitteln, Metallierungsmitteln und Alkylierungsmitteln und,
wobei die Polyisocyanatzusammensetzung eine organische Polyisocyanatfunktionalität von wenigstens 1,0 hat,
wobei das desaktivierte Amin unter Bedingungen gebildet wird, die die Bildung eines tertiären Amins der quaternären Ammoniumspezies verhindern, und die Reaktion zwischen desaktivierter Aminzusammensetzung und Polyisocyanatzusammensetzung so durchgeführt wird, daß ein Überschuß an Isocyanatgruppen über die Gesamtmenge an aktivem Wasserstoff in der desaktivierten Aminzusammensetzung von wenigstens 1,5:1,0 zur Verfügung gestellt wird.

2. Modifizierte Isocyanatzusammensetzung nach Anspruch 1, dadurch gekennzeichnet, daß der Polyether, dessen Endgruppen Aminogruppen sind, ein Molekulargewicht im Bereich von 500 bis 8000 hat und auf Ethylenoxid, Propylenoxid, Tetrahydrofuran oder deren Mischungen beruht.

3. Modifizierte Isocyanatzusammensetzung nach Anspruch 1, dadurch gekennzeichnet, daß die Polyisocyanatzusammensetzung ein Diphenylmethan-diisocyanat ist oder enthält.

4. Modifizierte Isocyanatzusammensetzung nach Anspruch 3, dadurch gekennzeichnet, daß die Polyisocyanatzusammensetzung 4,4'-Diphenylmethan-diisocyanat ist oder enthält.

5. Modifizierte Isocyanatzusammensetzung nach Anspruch 4, dadurch gekennzeichnet, daß die Polyisocyanatzusammensetzung eine Mischung von 4,4'-Diphenylmethan-diisocyanat mit den 2,4'- und 2,2'-Isomeren enthält.

6. Modifizierte Isocyanatzusammensetzung nach Anspruch 1, dadurch gekennzeichnet, daß das Verhältnis der Isocyanatgruppen zum gesamten aktiven Wasserstoff in der desaktivierten Aminzusammensetzung zumindest 5:1 beträgt.